# EUROPEAN PATENT SPECIFICATION

(11) **EP 2 219 513 B1**
(45) Date of publication and mention of the grant of the patent: **02.05.2012**
(21) Application number: 08849223.6
(22) Date of filing: 14.11.2008
(51) Int. Cl.: A61B 5/00, A61B 5/02

(54) **PULSE OXIMETRY METHOD**
PULSOXIMETRIEVERFAHREN
PROCÉDÉ D'OXYMÉTRIE PULSÉE

(30) Priority: 14.11.2007 US 987941 P
(43) Date of publication of application: 25.08.2010
(73) Proprietor: ConMed Corporation, Utica, NY 13502 (US)
(72) Inventor: WASSERMAN, Yoram, Haifa (IL); LOWERY, Guy, Russell, San Juan Capistrano CA 92675 (US)
(74) Representative: Palix, Stéphane
(86) International application number: PCT/US2008/083547
(87) International publication number: WO 2009/064979

(56) References cited:
- EP-A- 0 816 829
- US-A- 4 807 630
- US-A- 4 819 646
- US-A- 4 907 594
- US-A- 5 348 004
- US-A- 5 755 226
- US-A- 5 823 950
- US-A- 5 995 858
- US-A1- 2001 005 773
- US-A1- 2004 054 269
- US-A1- 2007 203 417
- US-B1- 6 997 879

## Description

### FIELD OF THE INVENTION

The invention relates generally to a method and apparatus for processing a pulsatile biometric signal. Specifically, the invention relates to pulse oximetry signal processing.

### BACKGROUND OF THE INVENTION

Pulse oximetry is a non-invasive diagnostic procedure for measuring the level of oxygen saturation in a patient's arterial blood. Pulse oximetry is based on the principle of passing light energy from at least two wavelengths to a light-absorptive physiologic medium, acquiring the reflected (or transmitted) emitted light in response to the light absorption, and calculating the oxygen saturation level from the acquired signals. By analyzing the color and/or changes in the reflected light, pulse oximeters are able to determine the heart beat (e.g., pulse rate) and/or level of oxygen in a patient. Pulse oximeters can include two components: a sensor attached to a patient's skin for acquiring signals and a processing unit for processing the acquired signals in order to determine the arterial blood oxygen saturation and pulse rate.

Conventional Reflective Pulse Oximetry (RPO) systems compute the level of oxygen saturation (e.g., SpO2) by determining a ratio of the AC_{RED}/DC_{RED} to AC_{IR}/DC_{IR}, where AC_{RED} is the AC component (e.g., the pulsating part) of the red wavelength detected light signal, DC_{RED} is the DC component (e.g., the average) of the red wavelength detected light signal, AC_{IR} is the AC component (e.g., the pulsating part) of the infrared wavelength detected light signal and DC_{IR} is the DC component (e.g., the average) of the infrared wavelength detected light signal. The DC component of a signal can be substantially greater than the AC component of a signal. For example, on a scale of 1 to 1,000,000, an AC component of a signal can be on the order of 1,000 units riding on top of a DC component of a signal on the order of 800,000 units.

A detector, such as a detector unit comprising a photodetector with a preamplifier unit, can only process a certain amount of light before it becomes saturated. Once a detector unit is saturated, it can no longer convert the photons from the light into electrical signals. Saturation can occur when a DC component of a signal is too high. An AC component of a signal can be hard to detect when the AC component is smaller as compared to the substantially larger DC component of the signal (e.g., dynamic range limitations). Increasing a level of an illumination of the light may not necessarily boost the AC component of the signal because increasing the level of illumination can lead to saturation of the detector by the DC component of the signal. Conventional RPO systems reduce the level of illumination to avoid detector saturation when encountered with a signal having a large DC component. Reducing the level of illumination, however, can make the AC component of the signal even smaller or can prevent the signal from penetrating deep enough to extract relevant data from the arteries, making it difficult to accurately determine AC/DC ratios.

Avoiding detector unit saturation can be difficult with living subjects having a high amount of pigmentation (e.g., subjects with dark skin) because the AC/DC ratio of a signal from the light reflected from the subjects can be much lower than normal. Avoiding saturation can also be difficult when there is a relatively high amount of biological tissue (e.g., fat, fluid, muscle, etc.) that contains a limited amount of arterial vessels. In that situation, the DC component can be larger than normal, and the AC component can be even smaller (e.g., 20 on a scale of 1 to 1,000,000). Avoiding saturation can also be difficult when the tissue under the sensor swells with fluid (e.g., edema which is associated with burn patients). The fluid and tissue can reflect more light, increasing the DC component of the signal, while decreasing an AC arterial signal content (e.g., AC component of the signal). Therefore, it can be difficult to avoid detector unit saturation while still having enough of an AC component (e.g., the pulsating part) of the signal to calculate accurately the pulse rate and the oxygen saturation in a patient having a high amount of pigmentation, high amount of biological tissue, edema, or any combination thereof. Techniques to avoid detector unit saturation can be beneficial if used in pulse oximetry systems used on patients having edema and can also allow for the placement of a sensor on a part of the body having a greater amount of pigmentation (e.g., on the forehead, ear or nose of the living subject) as compared to another part of the body (e.g., finger), on a part of the body having a high amount of biological tissue.

The invention concerns a pulse oximetry method and is defined in the appended claims. Document US2007/0203417 A1 discloses a pulse oximetry method for determining oxygen saturation level of a living subject comprising activating at least a first light source and a second light source to transmit light to the living subject during a time period; detecting a set of signals from light reflected from the living subject using a detector unit during the time period; filtering out a DC component of the set of signals to extract an AC component of the set of signals; processing the AC component to identify the location of a rising portion of the AC component; selecting signals from the set of signals that correspond to the rising portion of the AC component using the identified location; and calculating the level of oxygen saturation of the living subject using the selected signals.

However, document US2007/0203417 does not disclose using different sets of signals obtained from different time periods with different light sources activated for the identification of a rising portion of the AC component and for determining the oxygen saturation.

The disclosure herein concerns further methods and apparatus, aspects and features as follows.

### SUMMARY OF THE INVENTION

In one aspect, a method for processing a pulsatile signal of light reflected from a living subject is disclosed. The method can include the step of activating a light source to transmit light to the living subject during an accumulation time. The method can also include detecting at least one sample of the light reflected from the living subject using a detector unit and determining if the at least one sample of light approaches a saturation level of the detector unit. The method can include adjusting the accumulation time to prevent saturation of the detector unit if it has been determined (e.g., in the determining step) that the at least one sample of light has approached a saturation level of the detector unit.

In another aspect, a method for processing a pulsatile biometric signal can include detecting a first set of signals from a living source, where the first set of signals can include a pulsatile waveform and a constant component. The method can also include extracting the pulsatile waveform from the first set of signals and processing the pulsatile waveform to generate critical timing information. In some embodiments, the method can also include selecting from a second set of signals based on the critical timing information to calculate a biometric measurement. The second set of signals can include both the pulsatile waveform and the constant component.

According to the invention a method for processing a pulsatile signal of light reflected from a living subject includes activating at least a first light source and a second light source simultaneously to transmit light to a living subject during a first time period. The method further includes detecting a first set of signals from light reflected from the living subject using a detector unit and filtering out a DC component of the first set of signals (e.g., analog signal) to extract an AC component of the first set of signals. The method also includes processing the AC component to identify critical timing information, namely a rising portion of the AC component, that can be used to calculate a level of oxygen saturation of the living subject.

In other examples, any of the aspects above, or any apparatus or method described herein, can include one or more of the following features.

In some embodiments, a step of activating a light source can include transmitting light in at least one of a red frequency, infrared frequency, or any combination thereof. In some embodiments, a method for processing a pulsatile signal of light reflected from a living subject includes transmitting light in at least one of a red frequency, infrared frequency, second infrared frequency, or any combination thereof. In some embodiments, activating a light source to transmit light to a living subject during an accumulation period includes transmitting the light at a predetermined power level.

In some embodiments, determining whether at least one sample of light approaches a saturation level of a detector unit includes determining if the at least one sample of light has reached a threshold value. An accumulation time can adjusted to prevent saturation of a detector unit by lowering the accumulation time when at least one sample of light reaches a threshold value. In some embodiments, the threshold value is about 70% to about 85% of a saturation level of a detector unit. A step of lowering an accumulation time can include activating a light source (e.g., one or more LEDs in a pulse oximetry system) for a shorter period of time (e.g., shortening an activation period of the light source). In some embodiments, an accumulation time is adjusted by lowering the accumulation time until the accumulation time reaches a minimum value. In some embodiments, an accumulation time is 400 ms (e.g., initial accumulation time) and the minimum value for the activation time is 200 ms. A power level of the transmitted light (e.g., activated light source) can be adjusted to avoid saturation of a detector unit if the accumulation time has been lowered to a minimum value.

In some embodiments, a method for processing a pulsatile signal of light reflected from a living subject includes calculating the level of oxygen saturation of the living subject based on at least one sample of light (e.g., red light source, infrared light source, second infrared light source, or any combination thereof) reflected from the living subject during an accumulation period.

In some embodiments, a detector unit detects a first set of signals from light reflected from the living subject by outputting a first set of analog signals from light reflected from the living subject.

In some embodiments, at least a red light source and an infrared light source can be activated simultaneously to transmit light to a living subject during a first time period. In some embodiments, one or more of a red light source, first infrared light source, second infrared light source or any combination thereof can be activated individually or simultaneously during a first time period.

According to the invention, the method for processing a pulsatile signal of light reflected from a living subject includes activating a first light source to transmit light to a living subject during a second time period and detecting a second set of signals from the light reflected from the first light source during the second time period. The method also includes calculating the level of oxygen saturation of the living subject based on signals selected from the second set of signals. The signals selected from the second set of signals are those signals corresponding to the rising portion of the AC component. The rising portion of the AC component is determined by processing an AC component of signals detected during a first time period. According to the invention, the method is a pulse oximetry method.

In some embodiments, a set of signals (e.g., a first set of signals detected during a first time period when a first and second light source is simultaneously activated) can be filtered using at least one of a high pass filter or a low pass filter to filter out a DC component of the signals. In some embodiments, a first set of signals (e.g., analog signals) are filtered using a band pass filter to filter out the DC component of the signal and extract an AC component. In some embodiments, an AC component of a signal can be processed to determine a heart rate of the living subject.

In some embodiments, critical timing information is at least one of a heart rate of a living subject or a rising portion of the pulsatile waveform.

Other aspects and advantages of the invention can become apparent from the following drawings and description, all of which illustrate the principles of the invention, by way of example only.

### BRIEF DESCRIPTION OF THE DRAWINGS

The advantages of the invention described above, together with further advantages, may be better understood by referring to the following description taken in conjunction with the accompanying drawings. The drawings are not necessarily to scale, emphasis instead generally being placed upon illustrating the principles of the invention.

Figure 1 is a schematic of a pulse oximetry system, according to an illustrative embodiment.

Figure 2 depicts a process for detecting and processing pulsatile biometric signals of light, not forming part of the invention.

Figure 3 depicts a process for detecting and processing pulsatile biometric signals, according to an illustrative embodiment of the invention.

Figure 4 is a block diagram of a circuit for detecting and processing pulsatile biometric signals

### DETAILED DESCRIPTION OF THE INVENTION

While the illustrative embodiments disclosed herein are described in the context of pulse oximetry, the illustrative embodiments can be applied in other contexts that relate to signal processing of a pulsatile biometric signal. The illustrative embodiments can be applied to, for example, a reflective pulse oximetry system or a transmission pulse oximetry system.

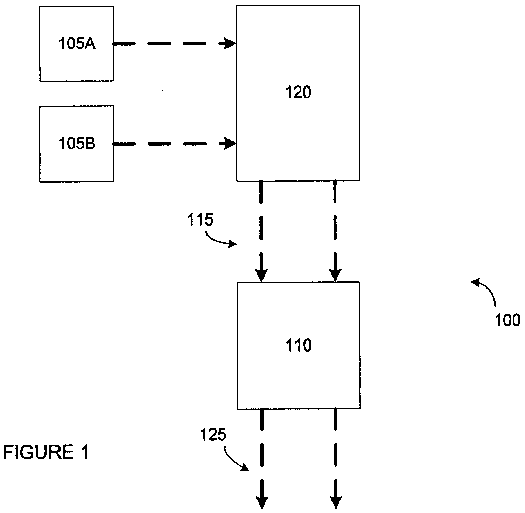

**Figure 1** is a schematic of a pulse oximetry system 100. A pulse oximetry system can detect samples using both red and infrared (IR) light at a plurality of times per second. The system can include a plurality of light sources 105A and 105B (e.g., a source of red light, a source of infrared light, etc.) that can be activated to transmit light for a period of time (e.g., an accumulation time). A detector unit 110 (e.g., photodetector and a preamplifier unit) can detect (e.g., sense or acquire) light reflected 115 (e.g., which can include the reflected red light or infrared light) from the living subject 120 and can convert the detected light (e.g., the photons from the reflected light) into an electric signal 125. The signal 125 can be processed to determine, for example, the heart rate of the subject and/or the level of oxygen saturation of the living subject 120.

For each sample detected by a detector unit 110 (e.g., photons of light detected by a photodetector), the detector unit 110 can accumulate charge for a fixed interval of time, where the accumulated charge is proportional to the amount of light detected. In some embodiments, the amount of time that the detector unit 110 accumulates charge is proportional to the amount of time that the light source lost or 105B transmits the light. Therefore, the sampling rate of the detector can be governed by the amount of time that the light source 105A or 105B transmits light (e.g., accumulation time). The output of the detector unit 110 can be then provided to an analog to digital converter, which can convert the detector output into a digital word (e.g., signal 125) that represents the amount of light received (e.g., reflected light 115), which can be processed. The system 100 can compute a level of oxygen saturation (e.g., SpO2) of the living subject 120 by determining a ratio of AC_{RED}/DC_{RED} to AC_{IR}/DC_{IR} where AC_{RED} is the value of the AC component (e.g., the pulsating part) of the reflected red light, DC_{RED} is the value of the DC component (e.g., the average) of the reflected red light, AC_{IR} is the value of the AC component (e.g., the pulsating part) of the reflected infrared light and DC_{IR} is the value of the DC component (e.g., the average) of the reflected infrared light.

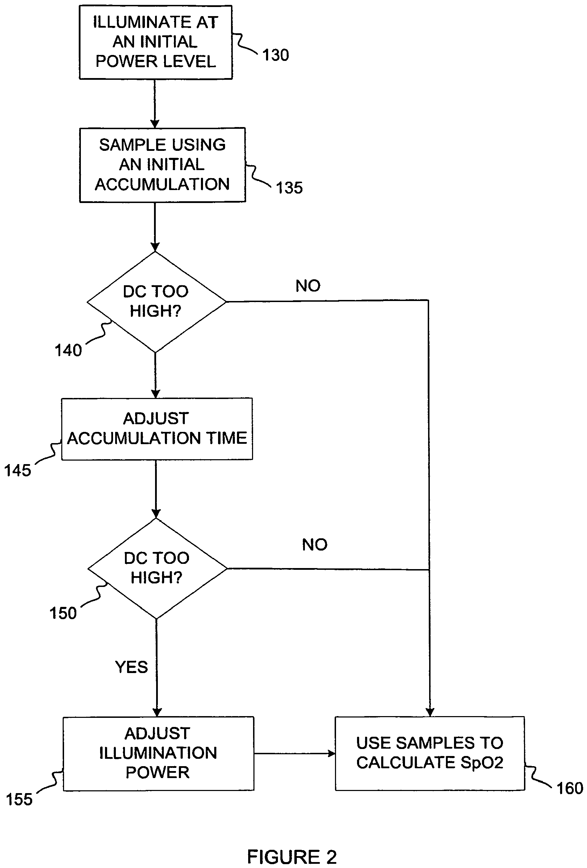

**Figure 2** depicts a process for detecting and processing pulsatile signals of light, not forming part of the invention. The process can be applied to, for example, a pulse oximetry system that can be reflectance pulse oximeter or a transmission pulse oximeter. The system can activate (e.g., illuminate) a light source (e.g., a LED) to transmit light to a living subject during an accumulation time. The system can use an initial power level (e.g., predetermined power level) for the light source (step 130). In some embodiments, the system includes a plurality of light sources (e.g., a red light source, an infrared light source, or any combination thereof etc) which can be activated to transmit light to the living subject. The light source can be initially activated/operated at a maximum power level intensity of an LED source. The system can detect at least one sample of the light reflected from a living subject (e.g., the patient's body) during an initial accumulation time using a detector unit (e.g., photodetector and an analog to digital converter circuit) (step 135). The signal (e.g., the light reflected from the patient, detected by the photodetector, outputted as an electrical analog signal which can be converted as a digital signal) includes a sum of an AC component and a DC component. The samples (e.g., or corresponding signals) of light can be processed to determined if the sample approaches a saturation level of the detector unit (step 140). If the sample approaches a saturation level of the detector, the system can adjust an accumulation time (e.g., the duration of each sample or the time that the light source is illuminated/activated) to maintain the light source at the initial power level without approaching saturation of the detector unit (step 145). The signal can approach a saturation level of the detector unit if a DC component of the signal is too high. If adjusting an accumulation time does not prevent the signal from approaching a saturation level of the detector unit (step 150), the system can continue to adjust the accumulation time until a minimum accumulation time value is reached (e.g., 200 ms). If the system has adjusted an accumulation time to the minimum value, the system can avoid detector saturation by reducing the power to the light source in steps (step 155). The sample(s) detected by the detector unit can be used to calculate, for example, an oxygen saturation level of a living subject (step 160).

A detector unit becomes saturated when the ADC converter (e.g., analog to digital converter circuit) of the detector unit reaches its maximum value. In some embodiments, the saturation level of a detector unit is 1,000,000 units. If a detector unit becomes saturated (e.g., the detector is no longer able to convert more units of photons of light into a digital signal), the detector unit outputs a constant value which represents the maximum value that the electric circuit is capable of providing. For example, if the saturation level of a detector is a predetermined number of units and the light detected exceeds this value, the detector will only output a constant value. The constant value can become the DC component of the signal but a saturated detector unit can not detect an AC component of the signal, therefore, a pulse rate and oxygen saturation level SpO2 can not be calculated.

The accumulation time can be the length of time that the light source is activated and illuminates/transmits light to the subject or the conversion time of the detector signal. To adjust an accumulation time, the system can vary the length of time that the light source illuminates/transmits light. In some embodiments, the system adjusts an accumulation time by adjusting a sampling time of the detector (e.g., adjusting the conversion time of the detector signal).

By way of example, a pulse oximeter system can activate light source to be illuminated (e.g., step 130) at a maximum power level and detect samples during an initial accumulation time (step 135). The accumulation time can be the time that the light source is activated (e.g., the period of time that the light source transmits light). The initial accumulation time can be predetermined (e.g., 400 micro seconds). The system can process the signal(s) to determine if the corresponding sample(s) detected by the detector (e.g., the integrated units of light or total units of light acquired during the accumulation time) approaches a saturation level of the detector unit (step 140). The system can determine if a signal approaches a saturation level of the detector unit by determining if the sample reaches a threshold value. The threshold value can establish a triggering event prompting the system to adjust the accumulation time (step 145) to avoid detector unit saturation. In some embodiments, if the signal received by the detector reaches a threshold value, the system responds by lowering the accumulation time of the detector. The triggering threshold value can be 70% to 85% of the saturation level of the detector. The system can adjust the accumulation time (step 145) to lower the accumulation time by shortening activation time of the light source (e.g., the amount of time that the light source transmits/illuminates light to the living subject). The system can continue to adjust the accumulation time (e.g., continue to lower the accumulation time) to avoid saturation until a minimum accumulation time value (e.g., 200 micro seconds) has been reached. By adjusting the accumulation time instead of adjusting a power level of the light source, the system can avoid saturation of the detector unit while simultaneously operating the light source at a desired power level (e.g., a maximum power intensity).

The light source can include an LED and an electronic driver for the LED. As more light is transmitted to a living subject, the deeper the light penetrates in the tissue of a living subject Transmitting more light (e.g., transmitting light at a higher power level) can allow the system to collect information from as much tissue volume as possible, thereby increasing accuracy of the measurements. In some embodiments, the system lowers an accumulation time so that the system can operate at a maximum power intensity level of the light source, thereby increasing accuracy of the measurements.

As described in **Figure 2****,** the accumulation time can be adjusted/lowered to avoid saturation of the detector (e.g., step 145). However, a minimum value for the accumulation time can be set so that the accuracy of the signal does not suffer. An accumulation time should be long enough to reduce the electronic noise to alter the accuracy (signal to noise ratio). If a noise level of a device is constant, a greater accumulation time results in a signal that is more valid (e.g., a better/greater signal to noise ratio). The minimum accumulation time value of a system can be defined according to the noise level of the system. By setting a minimum accumulation time value (e.g., the lowest possible accumulation time the system can handle before accuracy of the signal begins to suffer), the system can start off at a maximum power intensity level for the light source and incrementally lower the accumulation time to obtain the optimum working condition while avoiding saturation of the detector unit. If the accumulation time has been lowered to a minimum accumulation time value and lowering it further would cause the accuracy of the signal to suffer, the system can avoid detector saturation by lowering an intensity of the light source (e.g., a power level of the transmitted light) (e.g., step 155).

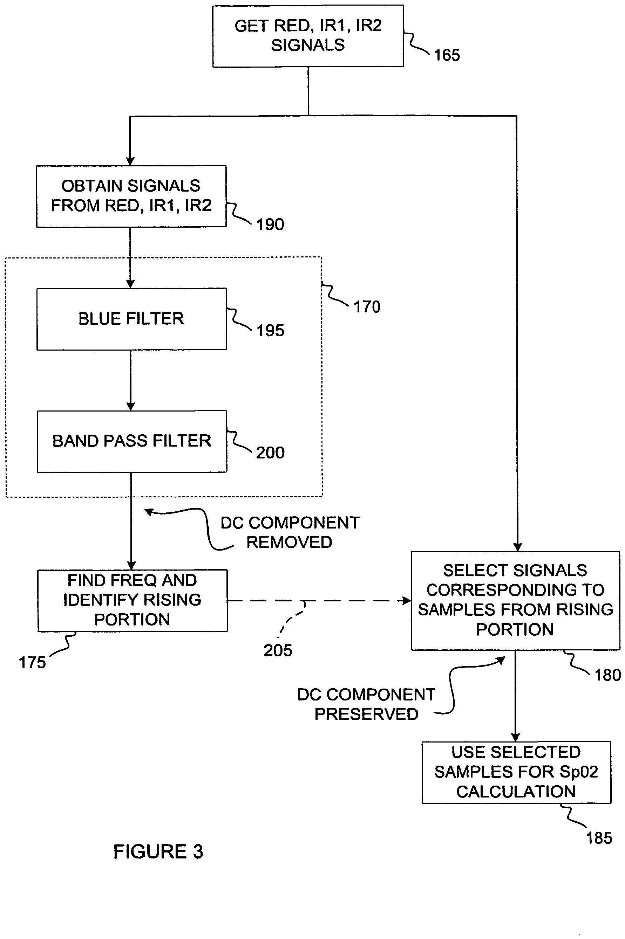

**Figure 3** depicts a process for detecting and processing a pulsatile biometric signal, according to an illustrative embodiment of the invention. The embodiments as described herein can be used to process a signal that includes a pulsatile waveform (e.g., pulsatile biometric signal) that is swamped by a background signal or noise (e.g., a signal comprising an AC component and a DC component where the DC component can be substantially larger than the AC component). The pulsatile waveform can be separately extracted to obtain, for example, critical timing information. The critical timing information can be used to process signals that include both the pulsatile waveform and the background signal/noise. The method for processing a pulsatile biometric signal includes detecting a first set of signals from a living source, where the first set of signals include a pulsatile waveform and a constant component. The pulsatile waveform can be extracted from the first set of signals and processed to generate critical timing information. The method can also include, for example, selecting from a second set of signals based on the critical timing information (e.g., heart rate of a living subject or a rising portion of the pulsatile waveform) to calculate a biometric measurement. The second set of signals can include both the pulsatile waveform and the constant component.

A pulse oximetry system can include a plurality of light sources (e.g., red light source, a first infrared light source, a second infrared light source, etc.) that can be individually or simultaneously activated to transmit light. In some embodiments, the plurality of light sources can be activated at different points in a cycle or at different time periods. A detector can detect samples (e.g., light reflected from the patient's body) of reflected light (step 165) during a first cycle, and another set of signals from a second cycle, etc. The detector can output analog signals from the detected samples of light that can be converted to digital signals. The signals detected during the different cycles are processed in two ways. A first set of signals (e.g., analog signals) are filtered to remove the DC component of the signal (step 170) and extract the AC component to obtain data relating to a pulsatile waveform (step 175). The pulsatile waveform is a cardiac/arterial waveform of a patient/subject and the data is (a heart rate and) the location of the rising portion of an arterial waveform. The data relating to the pulsatile waveform is used to process the data from another set of signals that include both the AC component and the DC component. The data relating to the pulsatile waveform is used to identify which one(s) of the signals from the second set of signals should be used (step 180) to determine a biometric measurement of a subject, namely the level of oxygen saturation (step 185). The set of signals used to detect/extract the AC component of the signal is detected in a different cycle than the set of signals where the AC component and DC component have been maintained and are used to calculate a biometric measurement of a subject.

In a pulse oximetry system, information from different wavelengths of light (e.g., a red light "R", infrared light "IR1", and a second infrared light "IR2") are used to calculate an oxygen level of a living subject. The pulse oximetry system can include a red light source, a first infrared light source and a second infrared light source. The light sources can be activated/operated separately or any combinations the individual light sources can be activated simultaneously (e.g., R, IR1, IR2, R+IR1, R+IR2, R+IR1+IR2, etc.) during different time periods/cycles. During each time period/cycle, a detector can detect reflected light from the living subject and output corresponding signals (e.g., sets of signals corresponding to each time period/cycle). Light sources can be activated every cycle, every second cycle, every third cycle or every fourth cycle. For example, a first light source (e.g., the red light source) can be activated during a first time period/cycle, a second light source (e.g., a first infrared light source) can be activated during a second time period/cycle, a third light source (e.g., a second infrared light source) can be activated during a third time period/cycle, and any combination of the light sources can be activated simultaneously during a fourth time period/cycle (e.g., red light source + first infrared light source, red light source + second infrared light source, red light source + first infrared light source + second infrared light source, etc.).

In conventional oximeters, the signal from the reflected light including the AC component and DC component is detected and converted to a digital form by the detector unit (e.g., the analog to digital converter (ADC) of the detector), and digital signal processing (e.g., digital filtering) is used to separate the DC component and AC component. However, by detecting an AC signal using a set of signals (e.g., signals detected at a point in the cycle when all the light sources are activated) to calculate critical timing information, a pulse oximetry system can generate greater accuracy of oxygen level measurements. The critical timing information from the AC component of the signal can be used to process signals acquired during other cycles to calculate, for example, oxygen level measurements. For example, signals from the other cycles (e.g., where only one or some of the plurality of light sources is on) can be used to calculate, for example, an oxygen level of a patient. Signals from the other cycles can include, for example, R, IR1, IR2, R+IR1, R+IR2, where R is the activated red light source, IR1 is an activated first infrared light source, and IR2 is the activated second infrared light source. These signals can be processed to better filter the noise (e.g., as we know already where the real pulses are), resulting in greater accuracy of oxygen level measurements.

The AC component is calculated/extracted using sets of signals (e.g., analog signals) of the reflected light detected in the cycle/time period when the plurality of light sources are simultaneously activated (step 190). Activating the plurality of light sources simultaneously can result in increased power. For example, where the light sources are simultaneously activated every fourth cycle, the extraction of the AC component (e.g., filtering to remove the DC component) can be done on sets of signals sampled for every fourth cycle. The system can execute a sample and hold operation to coincide with the desired cycle (e.g., the cycle when the light sources are simultaneously activated), and filter the output (e.g., analog signal) of the sample and hold to remove the DC component and extract out the AC component (e.g., step 170). Extracting out the AC component can include detecting the AC signal to eliminate preserving the ratio of the AC component of the signal to the background signal/DC component of the outputted signal.

To extract out the AC component of a signal (e.g., an analog signal), the signal can be filtered (e.g., by an analog filter) while it is in analog form (step 195 and step 200). The signal can be filtered to remove the DC component and only the AC component of the reflected light can be converted into a digital signal (e.g., where there is no need for high dynamic range from the ADC). Using an analog filter to filter out the DC component of the reflected light can result in an AC component that is stronger relative to the regular form (e.g., where AC component and the DC component has been maintained). It can be easier to filter out the DC component of a reflected light using an analog filter (e.g., filtering out the DC component while the signal is in analog form). The system may not acquire wavelength information, but the increased power in the signal results in a larger AC component of the signal (e.g., pulsating part (AC) of the signal). A larger AC signal results in a better/greater signal to noise ratio. Critical timing information, such as the pulse rate or the location of the pulse, can be detected more easily with a larger AC signal.

The system can filter out the DC component of the signal using an analog filter (e.g., an analog filter with a 6db at the pass points) while it is in analog form. The analog filter can be a bandpass filter (e.g., high and low pass filters) (step 200). Filtering out the DC component (step 200) can help extract the pulsatile waveform (e.g., the AC component of the signal). In some embodiments, the system is a pulse oximetry system and the pulsatile waveform is an arterial waveform. The system can use high pass filters and low pass filters to isolate/extract the specific pulsatile waveform. For example, if a typical heart rate has a frequency of approximately 30-300 beats per min, the system can use high pass filters and low pass filters to filter out portions of the signal that do not fall within the specified frequency range, therefore filtering out the portion of the signal that is not related to the arterial blood flow. In some embodiments, the high pass filter is used to filter out frequencies below about 0.7 Hz and the low pass filter is used to filter out frequencies above about 5.5 Hz, to extract a signal having frequencies within the range of about 0.7 Hz to about 5.5 Hz.

The system can use the filters (step 195 and step 200) to generate a large and precise arterial waveform, (e.g., instead of a very small AC component of the signal) which can be processed to determine the heart rate of a patient and determine critical timing information, such as the heart rate and/or locating the rising portion of the periodic signal (step 175). The step of processing a signal to determine the heart rate and/or locate the rising portion of the signal can be implemented using an algorithm such as the artificial waveform templates described in U.S. patent application US 2007/0203417 A1 entitled "Signal Processing for Pulse Oximetry" filed on September 28, 2006.

In some embodiments, the system blue filters (step 195) the analog signal outputted from the sensor of the detector. A pulse oximetry system can use a blue (step 195) to filter out the blue light to isolate/extract the pulsatile waveform (e.g., the arterial waveform). In some embodiments, the system uses a blue filter to filter out the portion of the signal that substantially corresponds to non-moving, non-arterial or venous blood and extract out the portion of the signal corresponding to arterial blood, which can be used to calculate the level of oxygen saturation.

Data relating to critical timing information, such as the heart rate and location of the rising portion (e.g., identified from the extracted AC component/AC signal in step 175), can be sent to the digital portion of the system (step 205). The critical timing information can be used to select/identify the signals (step 180) to be processed or analyzed to calculate an oxygen level in a patient. Signals that correspond to a rising portion of an arterial waveform (e.g., the AC component) are selected and used to calculate the oxygen saturation level (step 185). The AC to DC relationship of these signals has been maintained (e.g., the signal includes both the DC component and AC component). In some embodiments, the signals corresponding to the cycle where all the light sources are activated are used to calculate critical timing information while the signals from the other cycles (e.g., where only one/some but not all of the light sources are activated) are used to calculate biometric measurements, such as, oxygen saturation level of a patient. For example, if all the light sources are activated every fourth cycle, the signals from the first, second and third cycles can be used to calculate biometric measurements. The critical timing information can be used to enhance accuracy of the biometric measurements (e.g., Sp02) by determining which signals from the other cycles should be used in calculating biometric measurements.

By way of example, a method for processing a pulsatile signal of light reflected from a living subject can include simultaneously activating at least a first light source and a second light source (e.g., red light source and infrared light source) to transmit light to a living subject during a first time period. The method can include detecting a first set of signals from light reflected from the living subject using a detector unit (e.g., step 190) and filtering out a DC component of the first set of signals (e.g., using at least one of a high pass, low pass, or band pass filter) to extract an AC component of the first set of signals (e.g., step 170). Detecting a first set of signals can include outputting a first set of analog signals from light reflected from the living subject. The method can also include processing the AC component to identify a rising portion of the AC component (e.g., step 175) used to calculate a level of oxygen saturation of the living subject (e.g., steps 180 and 185). The AC component can also be processed to determine a heart rate of the living subject (e.g., step 175). The method can also include activating the first light source to transmit light to a living subject during a second time period and detecting a second set of signals from the light reflected from the first light source during the second time period. The level of oxygen saturation of the living subject can be calculated based on signals selected from the second set of signals corresponding to the rising portion of the AC component (e.g., step 180).

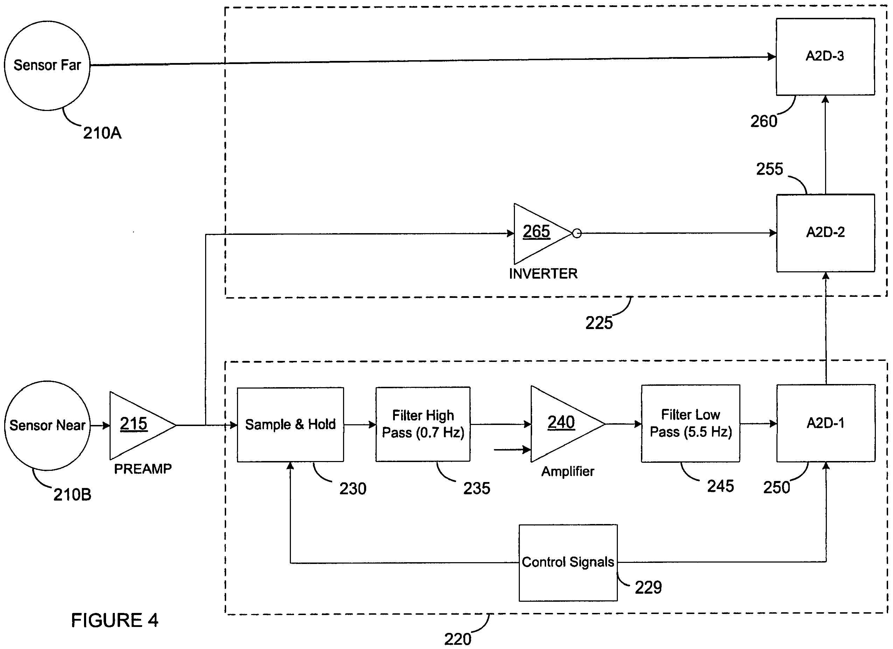

**Figure 4** is a block diagram of a circuit for detecting and processing pulsatile biometric signals. The circuit can include a sensor 210A or 210B for detecting samples of a biometric pulsatile signal (e.g., a photodetector that detects photons from reflected light from a patient). The circuit can generate signals (e.g., analog signals) based on the samples detected by the sensor 210A or 210B. The signals can include both an AC component (e.g., a pulsatile waveform) and a DC component. The circuit can include a preamplifier 215 that can amplify the signals from the sensor 210B. The circuit can include a first module 220 and a second module 225. The first module 220 can filter a selected set of the signals from the samples detected from the sensor 210B to filter out a DC component and isolate/extract an AC component of the signal. The AC component of the signal can be used to determine critical timing information (e.g., determine heart rate and/or identify the rising portion of an arterial waveform). The second module 225 of the circuit can process the signals from the samples detected by the sensor 210A or 210B, (e.g., to calculate an oxygen saturation level), which still have the AC component and the DC component of the signals preserved, using critical timing information generated from the first module 220. The circuit can also include analog to digital converters 250, 255 or 260.

In some embodiments, a preamplifier 215 filters the detector signal before digital conversion by converters 250 or 255. From the premplifier 215, the circuit can include two paths. A first path can go to an analog to digital converter 255, where the digital signal generated includes an AC component and a DC component which is later used to calculate, for example, an oxygen saturation level of a patient. The second path can be used to filter out a DC component of the signal, resulting in an AC component of an analog signal that can be used to obtain critical timing information, such as pulse rate calculations and a rising portion of a pulsatile waveform.

In some embodiments, only some of the signals from sensor 210B are used to detect/extract the AC component by the first module 220. For example, a plurality of light sources in a pulse oximetery system may not be simultaneously activated during every cycle. Any one or any combination of the plurality of light sources can be activated at different time periods in a sequence. In some embodiments, the analog filters 235 and 245 filter a continuous analog signal during one time period/cycle in the sequence. In some embodiments, the AC signal is only detected (e.g., the DC component filtered out) and the pulse rate is calculated on signals during the cycles/time periods/sequence when more than one light source is activated simultaneously. A controller 229 can control a sample and hold unit 230 to operate on the analog signal from the detector only during the cycle/time period where all or more than one of the light sources are simultaneously activated (e.g., cycle when a red light source, a first infrared light source and a second infrared light source is activated simultaneously).

In some embodiments, the plurality of light sources (e.g., red light source, first infrared light source, second infrared light source, etc.) in a pulse oximetry system are simultaneously activated every fourth cycle and a sample and hold can operate on the analog signal every fourth cycle. The analog signal from this fourth cycle can be processed by the first module 220 and filtered to remove the DC component, amplified using amplifier 240 and converted to a digital form by converter 260 (e.g., A2D-1). The AC component of the signal can be used to calculate, for example, the pulse rate/location of a living subject. The analog signals from the other cycles (e.g., R, IR1, IR2) which still include the AC component and the DC component can be used to calculate, for example, an oxygen saturation level of a living subject. The analog signals from these other cycles (e.g., where more than one LED is not activated) can be converted to a digital form (e.g., with converter 255). In some embodiments, the signal from each light source during each "on period" (e.g., the period of time when each LED is individually activated) is converted to a digital form by converters 255 and 260 in the second module 225. These digital signals reflect information from each individual light source and reflect information from the different wavelengths of light (e.g., red, first infrared, second infrared, etc.) which can be used to calculate the oxygen (e.g., SpO2) level.

By way of example, a red light source, first infrared light source and second light infrared source can be activated according to the following cycles/sequences: R, IR1, IR2, IR1+IR2+R, R, IR1, IR2, etc. where R is the red light source, IR1 is a first infrared light source, and IR2 is a second infrared light source. In some embodiments, only the signals from the detector during the cycle where all the light sources are simultaneously activated are filtered analog filters 235 and 245 (e.g., every fourth cycle/time period/sequence). To reconstruct an analog signal from the fourth sequence, a controller 229 can send a "sample signal" to the sample and hold unit 230 during the fourth sequence to direct the sample and hold unit 230 to sample the detector output during this time period. The output of the sample and hold unit 230 can remain with this value (e.g., hold) during the other sequences (e.g., first sequence where red light is activated, the second sequence when the first infrared source is activated and the third sequence when the second infrared source is activated) when the other light sources are individually activated. The result is the sample and hold 230 can output an analog signal that corresponds to the time periods of the sequence where all the light sources are activated (e.g., fourth sequence). This analog signal can be now processed by the filters 235 and 245 and amplifier 235 of the second module 220 and converted into a digital form by converter 250.

The selected set of analog signals can be sent to a first filter 235 in the first module 220, which can be a high pass filter. In some embodiments, the high pass filter allows signals of a frequency greater than approximately 0.7 Hz to pass, filtering out signals having a frequency lower than approximately 0.7 Hz. The circuit can also include an amplifier 240 that amplifies the filtered signal and correct for any offsets. The electronics from the circuit (e.g., amplification from preamp 215 and filtering from filter 235) can add another DC component to the signal (e.g., offsets or voltage bias) that can be removed using amplifier 240 with an inverted input that adjusts the DC voltage to the level needed to eliminate the DC component that is removed by the first module 220. The circuit can also include a second filter 245. In some embodiments the second filter 245 is a low pass filter that filters out signals having a frequency greater than approximately 5.5. Hz. The signal outputted/generated by the first module of the circuit, therefore, is the AC component of the signal (e.g., the pulsatile waveform resulting from the DC component having been filtered out) that is converted into a digital signal by converter 250. In some embodiments, the resulting AC component of the signal has been amplified and filtered to generate a large and precise arterial waveform. The AC component of the signal (e.g., the pulsatile waveform) can be used to generate critical timing information (e.g., rising portion of an arterial waveform).

The second module 225 of the circuit processes the analog signals from the samples detected by the sensor 210A or 210B which can be converted into digital signals using converters 255 and 260. These signals still have the AC component and DC component maintained/preserved. These signals can be the signals from, for example, the cycles where the all the light sources are not simultaneously activated. In some embodiments, the signals processed by the second module are not the signals that are filtered and processed by the first module to generate an AC only component. In some embodiments, the second module includes an inverter 265. An amplifier (e.g., preamp 215) can act as a converter and an inverter 265 can invert the input signal from the sensor 210B and ADC converter 255. The signals from the sensor 210A or 210B can be processed by the circuit (e.g., second module 225) to determine, for example, a heart rate and/or oxygen saturation level of a subject. The critical timing information from the AC component of the signal generated by the first module 220 can be used to select which of the signals (e.g., signals from converter 255 or 260) from the second module 225 will be processed, for example, to calculate a level of oxygen saturation in a subject.

In some embodiments, the system has two sensors 210A and 210B (e.g., photodetectors) that detects light reflected from different locations, which can enhance the accuracy of the measurements (e.g., measurement of level of oxygen saturation). In this embodiment, the system includes a sensor near 210B and sensor far 210A that detects reflected light at different depths/distances to ensure that readings are not distorted by, for example, a vein located close to one of the sensors.

While the invention has been particularly shown and described with reference to specific illustrative embodiments, it should be understood that various changes in form and detail may be made without departing from the scope of the invention.

## Claims

1. A pulse oximetry method for determining oxygen saturation level of a living subject comprising:
• activating at least a first light source (105A) and a second light source (105B) simultaneously to transmit light to the living subject during a first time period;
• detecting a first set of signals from light reflected (115) from the living subject using a detector unit during the first time period;
• filtering out a DC component of the first set of signals to extract an AC component of the first set of signals; and
• processing the AC component to identify the location of a rising portion of the AC component;
• activating the first light source (105A) to transmit light to a living subject during a second time period different from the first time period;
• detecting a second set of signals from the light reflected from the first light source (105A) during the second time period;
• selecting signals from the second set of signals that correspond to the rising portion of the AC component using the identified location; and
• calculating the level of oxygen saturation of the living subject using the selected signals.

2. The method of claim 1, wherein detecting the first set of signals comprises outputting a first set of analog signals from light reflected from the living subject.

3. The method of claim 1, wherein activating at least a first light source (105A) and a second light source (105B) simultaneously comprises activating at least a red light source and an infrared light source simultaneously to transmit light to the living subject during the first time period.

4. The method of claim 1, wherein processing further comprises determining a heart rate of the living subject.

5. The method of claim 1, wherein filtering comprises filtering the first set of signals using at least one of a high pass filter or a low pass filter.

6. The method of claim 1, wherein filtering comprises filtering the first set of signals using a band pass filter to filter out the DC component and extract the AC component.

## Patentansprüche

1. Pulsoxymetrieverfahren zur Bestimmung des Sauerstoffsättigungsgrads eines Lebewesens, Folgendes umfassend:
• Gleichzeitiges Aktivieren mindestens einer ersten Lichtquelle (105A) und einer zweiten Lichtquelle (105B), um während eines ersten Zeitraums Licht zum Lebewesen zu übertragen;
• Erfassen eines ersten Satzes von Signalen während des ersten Zeitraums aus dem vom Lebewesen reflektierten Licht (115) unter Verwendung einer Detektoreinheit;
• Herausfiltern einer DC-Komponente aus dem ersten Satz von Signalen, um eine AC-Komponente aus dem ersten Satz von Signalen zu extrahieren; und
• Verarbeiten der AC-Komponente, um die Stelle eines ansteigenden Teils der AC-Komponente ausfindig zu machen;
• Aktivieren der ersten Lichtquelle (105A), um während eines zweiten Zeitraums, der sich vom ersten Zeitraum unterscheidet, Licht zu einem Lebewesen zu übertragen;
• Erfassen eines zweiten Satzes von Signalen während des zweiten Zeitraums aus dem von der ersten Lichtquelle (105A) reflektierten Licht;
• Auswählen von Signalen aus dem zweiten Satz von Signalen, die dem ansteigenden Teil der AC-Komponente entsprechen, unter Verwendung der ausfindig gemachten Stelle; und
• Berechnen des Grads an Sauerstoffsättigung des Lebewesens unter Verwendung der ausgewählten Signale.

2. Verfahren nach Anspruch 1, wobei das Erfassen des ersten Satzes von Signalen umfasst, einen ersten Satz analoger Signale aus dem vom Lebewesen reflektierten Licht auszugeben.

3. Verfahren nach Anspruch 1, wobei das gleichzeitige Aktivieren mindestens einer ersten Lichtquelle (105A) und einer zweiten Lichtquelle (105B) umfasst, mindestens eine Rotlichtquelle und eine Infrarotlichtquelle gleichzeitig zu aktivieren, um während des ersten Zeitraums Licht zum Lebewesen zu übertragen.

4. Verfahren nach Anspruch 1, wobei das Verfahren darüber hinaus umfasst, eine Herzfrequenz des Lebewesens zu bestimmen.

5. Verfahren nach Anspruch 1, wobei das Filtern umfasst, den ersten Satz von Signalen unter Verwendung eines Hochpassfilters und/oder eines Tiefpassfilters zu filtern.

6. Verfahren nach Anspruch 1, wobei das Filtern umfasst, den ersten Satz von Signalen unter Verwendung eines Bandpassfilters zu filtern, um die DC-Komponente herauszufiltern und die AC-Komponente zu extrahieren.

## Revendications

1. Procédé d'oxymétrie par impulsions pour déterminer le niveau de saturation en oxygène d'un sujet vivant, consistant à :
- activer au moins une première source lumineuse (105A) et une seconde source lumineuse (105B) simultanément pour la transmission d'une lumière vers le sujet vivant pendant une première période de temps ;
- détecter un premier ensemble de signaux provenant d'une lumière réfléchie (115) provenant du sujet vivant en utilisant une unité de détection pendant la première période de temps ;
- filtrer une composante de courant continu DC du premier ensemble de signaux pour extraire une composante de courant alternatif AC du premier ensemble de signaux ; et
- traiter la composante AC pour identifier l'emplacement d'une partie montante de la composante AC ;
- activer la première source lumineuse (105A) pour transmettre une lumière à un sujet vivant pendant une seconde période de temps différente de la première période de temps ;
- détecter un second ensemble de signaux à partir de la lumière réfléchie provenant de la première source lumineuse (105A) pendant la seconde période de temps ;
- sélectionner des signaux provenant du second ensemble de signaux qui correspondent à la partie montante de la composante AC en utilisant l'emplacement identifié ; et
- calculer le niveau de saturation en oxygène du sujet vivant en utilisant les signaux sélectionnés.

2. Procédé selon la revendication 1, dans lequel la détection du premier ensemble de signaux comprend l'émission d'un premier ensemble de signaux analogiques à partir d'une lumière réfléchie à partir du sujet vivant.

3. Procédé selon la revendication 1, dans lequel l'activation simultanément d'au moins une première source lumineuses (105A) et d'une seconde source lumineuse (105B) comprend l'activation simultanément d'au moins une source de lumière rouge et une source de lumière infrarouge pour transmettre une lumière au sujet vivant pendant la première période de temps.

4. Procédé selon la revendication 1, dans lequel le traitement comprend de plus la détermination du rythme cardiaque du sujet vivant.

5. Procédé selon la revendication 1, dans lequel la filtration comprend la filtration du premier ensemble de signaux en utilisant au moins un filtre passe haut ou un filtre passe bas.

6. Procédé selon la revendication 1, dans lequel la filtration comprend la filtration du premier ensemble de signaux en utilisant un filtre passe bande pour filtrer la composante DC et extraire la composante AC.
